# EUROPEAN PATENT APPLICATION

(11) **EP 4 609 898 A1**
(43) Date of publication of application: **03.09.2025**
(21) Application number: 24160331.5
(22) Date of filing: 28.02.2024
(51) Int. Cl.: A61M 5/315

(54) **METHOD OF MANUFACTURING A LAMINATED STOPPER, POLYMER BLENDS FOR SAME AND STOPPERS MADE THEREFROM**

(71) Applicant: Becton, Dickinson and Company, Franklin Lakes, NJ 07417-1880 (US)
(72) Inventor: RODRIGUEZ SAN JUAN, Nestor, Hamburg, NJ07419 (US); FORCINITI, Leandro, 410 Wharton, NJ07885 (US); FLIPPE, Marc, 38640 CLAIX (FR); PERRIN, Eloïse, 38320 Eybens (FR); RODRIGUEZ, Gumersindo, Sumter, SC29154 (US)
(74) Representative: Germain Maureau

(57) **Abstract**

The invention relates, in general, to various methods to manufacture and/or design stoppers, in particular laminated stoppers for medical applications, as well as various polymer blends and design considerations to be considered when manufacturing such stoppers. In one embodiment, the present invention relates to a syringe stopper formed from a material having an increased rubber modulus and/or a compression ratio, as defined below, that yields a partially laminated stopper with only one or more desired sealing services being laminated (e.g. a stopper with, for example, three ribs but only one or two thereof being a laminated sealing rib) or a fully laminated stopper with all sealing services being laminated (e.g. a stopper with, for example, three ribs where all three ribs are laminated sealing ribs) sealing surfaces, stopper with suitable contact pressures (Aᵣₑₐₗ/Aₒ is greater than 0.42) so as to realize total sealing in a wide variety of applications.

## Description

### BACKGROUND OF THE INVENTION

### Field of the Invention

The invention relates, in general, to various methods to manufacture and/or design stoppers, in particular laminated stoppers for medical applications, as well as various polymer blends and design considerations to be considered when manufacturing such stoppers.

### Description of the Related Art

Current syringe designs can typically be categorized as two-piece syringes or three-piece syringes. A typical three-piece syringe includes a tubular barrel having an access opening formed at one end, and a smaller discharge opening formed at the opposing end. The lead end of an elongated plunger is received within the access opening of the barrel so as to be slidable within the barrel. Attached to the lead end of the plunger is a flexible sealing member or stopper that snugly seals against the interior surface of the barrel. A needle, a threaded member, or a non-threaded member is usually attached to the discharge opening on the barrel. The needle can be used to penetrate a surface while the threaded member can be used to attach the syringe to another medical device, such as a catheter. The flexible stopper is usually manufactured from an elastomeric material, such as a rubber or a cross-linked or thermoplastic elastomer or an elastomer-plastic blend.

A two-piece syringe, on the other hand, includes a "stopper" that is in the form of a rigid sealing disc, also known as a plunger rod head. It is typically made of the same rigid plastic as the rest of plunger rod. The sealing force in a two-piece syringe comes from a barrel (formed from a glass or suitable polymer) that may or may not deform around the rigid plunger rod head. Previously, it has been identified that the surface energy of the counter surface, the syringe barrel (be it glass or plastic), is the main parameter on the gliding performance of the barrel. In a fully laminated system, there is almost not adhesion between the film (e.g., PTFE or UHMWPE) and the one or more counter surfaces. This is mainly due to two aspects, first the surface energy between the glass-polymer film or polymer film-polymer barrel are dissimilar, and second the film hardness is so high that the film has a low conforming contact area as will be explained later. Consequently, the surface energy of the one or more counter surfaces presents no significant impact on the gliding performance.

It was previously believed that the main contribution of the surface energy is to the leakage rate through the interface by contributing to the net differential pressure across the interface in the form of Laplace pressure. To date, no one has yet to achieve a laminated stopper that achieves a leakage rate based on the percolation threshold between the material used for the laminated portion of such a laminated stopper and either a glass or polymer counter surface. Therefore, the previous contributions relating to Laplace pressure are irrelevant in a system where the percolation threshold is achieved as explained below.

Current two-piece and three-piece syringe designs suffer from various deficiencies. For instance, three-piece syringes that include an elastomeric or rubber stopper do not have enough contact pressure at the sealing surface to conform to one another, thereby resulting in leakage in detriment of container closure integrity. The main reason for such deficiency is the fact that the contact pressures achieved are not enough to elasto-plastically deform a laminated surface to fully seal/conform to the counter surface (e.g., a glass or polymer counter surface). Those various passive contact pressures can be as high as approximately 300 psi (approximate 2 MPa), which are significantly smaller than the hardness on indentation of the material used for the laminating film which is above 1450 psi or 10 MPa.

Furthermore, after initial assembly of a syringe, the contact pressures at the sealing surfaces decay due to stress relaxation and when the Container Closure Integrity (CCI) of the syringe is challenged during either quality control testing and/or due to differential pressures during shipping. As such, frequently the stopper moves from its original position and the need to re-conform to the surface rises again but now with significantly lower contact pressures.

The advent of more accurate measurements of leakage through interfaces has revealed gas leakage rates previously not detected in laminated systems through a stopper-barrel interface that are significantly larger than the permeability of the film-elastomer bulk. While not wishing to be bound to any one theory, the leakage rates should be basically on the order of the permeability of the film-elastomer bulk only (e.g., for a halogenated isobutylene rubber with a thickness of approximately 0.6 mm the air permeability if of the order of 1⁻⁹ mm³ STP/mm-mm²-cmHg or less).

Therefore, a need exists for a sealing element for a syringe that has sufficient contact pressure between the stopper and the barrel such that the one or more sealing film layers elasto-plastically deform to achieve full sealing while still having acceptable activation and gliding performance (e.g., an activation and gliding performance below 15 N). This requirement must be fulfilled even after the sealing contact pressures relax during shelf life and in the event of possible stopper-barrel relative displacements. In another instance, the intent of this invention is to obtain a stopper-barrel systems with no need for any lubrication layer at the interface via the use of a silicone oil type lubricant or one or more additional surface chemical modifications.

Thus, the present invention illustrates that in order to achieve proper sealing (i.e., percolation threshold) between an inner surface of a syringe, or a syringe barrel, and a stopper surface, the contact pressure that any one or more sealing ribs of the stopper must seek to achieve contact pressures above or very close to the indentation hardness modulus (otherwise referred to as the yield of the stopper's film material) of a partially laminated, or fully laminated, stopper sealing surface.

Based on this, a need exists for a syringe stopper having a desirable geometry that realizes a combination of a reduction in the contact area with an inner surface of a syringe barrel (or other article to be sealed) thereby yielding a reduction in the radius of contact and increase in contact pressure, a high modulus of the elastomer substrate, and a compression ratio between the stopper and syringe barrel - so as to achieve a stopper having a more desirable yield of a sealing film material. A further need exists for a stopper formed from a material that has an increased rubber modulus thereby yielding a stopper (regardless of whether such stopper is partially or fully laminated) with suitable contact pressures so as to realize total sealing in a wide variety of applications.

### SUMMARY OF THE INVENTION

The invention relates, in general, to various methods to manufacture and/or design stoppers, in particular laminated stoppers for medical applications, as well as various polymer blends and design considerations to be considered when manufacturing such stoppers.

In one embodiment, the present invention relates to a syringe stopper formed from a material having an increased rubber modulus and/or a compression ratio, as defined below, that yields a partially laminated stopper with only one or more desired sealing services being laminated (e.g. a stopper with, for example, three ribs but only one or two thereof being a laminated sealing rib) or a fully laminated stopper with all sealing services being laminated (e.g. a stopper with, for example, three ribs where all three ribs are laminated sealing ribs) sealing surfaces, stopper with suitable contact pressures to achieve an Aᵣₑₐₗ/Aₒ greater than 0.42 and so as to realize total sealing in a wide variety of applications, where Aᵣₑₐₗ is the real contact area at the sealing surfaces measured at high magnification (for example, a magnification equal to or greater than 500X) and Aₒ is the nominal area of the sealing surfaces measured at very low magnification (for example, a magnification of less than 20X).

While not wishing to be bound to any one theory and/or outcome, it is believed that the present invention will work adequately on bare (clean) glass, boro- or alumina-silicate glass barrels (typically a water contact angle (WCA) of less than 60 degrees), or polymer barrels (typically a water contact angle (WCA) of greater than 60 degrees). The WCA is defined as the angle measured by sessile drop, using ultra purified water (typically equal or larger than 18.2 Mohms and a Total Organic Carbon (TOC) of less than 6 ppb) with a volume of 1.0 µL ± 0.1 µL and averaging over at least 10 measurements in different locations of the corresponding surface. Glass and polymer barrels may have different WCAs depending on the state of the surface, for example when contaminated with silicone oil the WCA is typically greater than 60 degrees when the glass or polymer surface have adsorbed layers of hydrocarbons present in the environment (e.g., pollution for auto motor emissions in the air).

In one embodiment, the present invention relates to a syringe stopper formed from a material having an increased rubber modulus and/or a compression ratio, as defined below, that yields a partially laminated stopper with only one or more desired sealing services being laminated (e.g. a stopper with, for example, three ribs but only one or two thereof being a laminated sealing rib) or a fully laminated stopper with all sealing services being laminated (e.g. a stopper with, for example, three ribs where all three ribs are laminated sealing ribs) sealing surfaces, stopper with suitable contact pressures in order to achieve an Aᵣₑₐₗ/Aₒ greater than 0.42 so as to realize total sealing in a wide variety of applications.

According to one aspect, the present invention is directed to a stopper comprising: a stopper body formed from a polymeric material; and at least one sealing rib formed on at least one portion of the stopper body, wherein the polymeric material is selected from one or more polyolefins, one or more polyamides, one or more polyesters, one or more polystyrenes, one or more polyurethanes, one or more polycarbonates, one or more acrylonitrile-butadiene-styrenes, one or more fluoropolymers, one or more polyacrylates, one or more elastomeric rubbers, one or more polyisoprenes, one or more polyisobutylene, one or more polybutadienes, one or more chloroprene rubbers, one or more butyl rubbers, one or more halogenated butyl rubbers, one or more styrene-butadiene rubbers, one or more nitrile rubbers, one or more hydrogenated nitrile rubbers, natural rubber, one or more ethylene propylene rubbers, one or more ethylene propylene diene rubbers, or blends of any two or more thereof where the polymeric material, or the blend of polymeric materials, has a Young's modulus in a range of about 5 MPa to about 10 MPa and a Shore A hardness in a range of about 50 to about 85.

In another embodiment, the present invention is directed to a stopper that is formed from a polymeric material that has a Young's modulus in a range of about 6 MPa to about 9 MPa and a Shore A hardness in a range of about 55 to about 80, or even about 60 to about 75. In still another embodiment, the present invention is directed to a stopper that is formed from a polymeric material that has a Mooney viscosity of greater than about 40 before curing. In still another embodiment, the present invention is directed to a stopper that is formed from a polymeric material that has a stress relaxation better than about 30 percent at room temperature (RT or 25 °C in a period of 24 hours. In still another embodiment, the present invention is directed to a stopper that is formed from a polymeric material that further comprises at least one filler present at an amount in a range of about 40 phr to about 90 phr, or more preferably in a range of about 60 phr to about 70 phr. In still another embodiment, the present invention is directed to a stopper that is formed from a polymeric material that has a filler is selected from a silica filler. In still another embodiment, the present invention is directed to a stopper that is formed from a polymeric material that is selected from a bromo butyl rubber.

In still another embodiment, the present invention is directed to a stopper that has a stopper body that has an Aᵣₑₐₗ/Aₒ in a range of about 0.42 to about 0.70 at least one of its sealing contact surfaces and/or sealing ribs - where Aᵣₑₐₗ is the actual contact area measured at very high magnification (X greater than 500) and Aₒ is the nominal contact area observed at low magnification (X less than 20 for example using a 8X magnifier). Previously the art defined and only considered Aₒ and no one consider Aᵣₑₐₗ at all, nor was any relationship, or even ratio, between Aᵣₑₐₗ and Aₒ considered. In still another embodiment, the present invention is directed to a stopper where the stopper body further comprises at least two sealing ribs formed thereon. In still another embodiment, the present invention is directed to a stopper where at least one of the ribs is a sealing rib and is laminated with at least one lamination layer. In still another embodiment, the present invention is directed to a stopper where all the ribs, sealing or otherwise, are laminated with at least one lamination layer. In still another embodiment, the present invention is directed to a stopper where all the ribs, regardless of whether or not such ribs are sealing ribs, and the stopper body are laminated with at least one lamination layer. In still another embodiment, the present invention is directed to a stopper where any lamination layer is formed from at least one compound selected form densified expanded polytetrafluoroethylene (ePTFE), polytetrafluoroethylene (PTFE), fluorinated ethylene propylene (FEP), polyethylene (e.g., UHMWPE), polypropylene (e.g., syndiotactic-PP), polyvinylidene fluoride, polyvinylfluoride, perfluoropropylevinylether, a perfluoroalkoxy polymer, polysulfones, and copolymers and combinations thereof. In still another embodiment, the present invention is directed to a stopper, or any other sealing device or structure, where at least one lamination layer is formed from at least one layer of polytetrafluoroethylene (PTFE), UHMWPE, or polysulfone. In still another embodiment, the present invention is directed to a stopper where the surface RMS (root mean square) roughness of the polymeric material that is used to form the stopper of the present invention is in a range of about 0.0001 microns to about 4 microns as measured at 50X via optical interferometry (where the field of view is approximately 100 × 100 µm²).

In still another aspect, the present invention is directed to a syringe comprising: a syringe barrel; a plunger, wherein the plunger is designed to fit within a syringe barrel; and the stopper has a compression ratio between about 1 percent and about 7.4 percent, or even between about 2 percent and 7 percent according to any of the embodiments disclosed herein. In another aspect, the present invention is directed to a stopper that is adapted for attachment to one end of a plunger so as act as a seal for a syringe barrel. In another aspect, the present invention is directed to a stopper with a compression ratio (CR) that is defined as the initial value before assembly such that CR = 100% X (R_{free stopper OD} - R_{barrel ID}) / R_{barrel ID}.

In still another embodiment, the present invention is directed to a sealing device comprising: a sealing body formed from a polymeric material; and at least one sealing structure and/or or sealing rib formed on at least one portion of the sealing body, wherein the polymeric material is selected from one or more polyolefins, one or more polyamides, one or more polyesters, one or more polystyrenes, one or more polyurethanes, one or more polycarbonates, one or more acrylonitrile-butadiene-styrenes, one or more fluoropolymers, one or more polyacrylates, one or more elastomeric rubbers, one or more polyisoprenes, one or more polyisobutylene, one or more polybutadienes, one or more chloroprene rubbers, one or more butyl rubbers, one or more halogenated butyl rubbers, one or more styrene-butadiene rubbers, one or more nitrile rubbers, one or more hydrogenated nitrile rubbers, natural rubber, one or more ethylene propylene rubbers, one or more ethylene propylene diene rubbers, or blends of any two or more thereof where the polymeric material, or the blend of polymeric materials, has a Young's modulus in a range of about 5 MPa to about 10 MPa and a Shore A hardness in a range of about 50 to 85, that can be utilized to seal any type of medical device and/or other device needing an adequate sealing device.
Clause 1: A stopper comprising: a stopper body formed from a polymeric material; and at least one sealing rib formed on at least one portion of the stopper body, wherein the polymeric material is selected from one or more polyolefins, one or more polyamides, one or more polyesters, one or more polystyrenes, one or more polyurethanes, one or more polycarbonates, one or more acrylonitrile-butadiene-styrenes, one or more fluoropolymers, one or more polyacrylates, one or more elastomeric rubbers, one or more polyisoprenes, one or more polyisobutylene, one or more polybutadienes, one or more chloroprene rubbers, one or more butyl rubbers, one or more halogenated butyl rubbers, one or more styrene-butadiene rubbers, one or more nitrile rubbers, one or more hydrogenated nitrile rubbers, natural rubber, one or more ethylene propylene rubbers, one or more ethylene propylene diene rubbers, or blends of any two or more thereof where the polymeric material, or the blend of polymeric materials, has a Young's modulus in a range of about 5 MPa to about 10 MPa and a Shore A hardness in a range of about 50 to about 85.
Clause 2: The stopper of clause 1, wherein the polymeric material has a Young's modulus in a range of about 5 MPa to about 8 MPa and a Shore A hardness in a range of about 60 to about 70.
Clause 3: The stopper of any of clauses 1 or 2, wherein the polymeric material has a Mooney viscosity of greater than about 40, before curing.
Clause 4: The stopper of any of clauses 1 to 3, wherein the polymeric material has a stress relaxation better than about 30 percent at room temperature (RT) in a period of 24 hours and a compression ratio (CR) under 25 percent (as determined by ASTM D4065).
Clause 5: The stopper of any of clauses 1 to 4, wherein the polymeric material further comprises at least one filler present at an amount in a range of about 50 phr to about 90 phr, or more preferably in a range of about 60 phr to about 70 phr.
Clause 6: The stopper of clause 5, wherein the filler is selected from any one or more suitable reinforcement fillers with a particle having a surface area greater than about 100 m²/g, or preferably a silica filler having a surface area greater than 100 m²/g.
Clause 7: The stopper of any of clauses 1 to 6, wherein the polymeric material is selected from a halogenated isobutylene-isoprene copolymer rubber.
Clause 8: The stopper of any of clauses 1 to 6, wherein the polymeric material is selected from a styrene-butadiene, a high-cis butadiene, an ethylene propylene diene terpolymer, or a brominated isobutylene paramethyl-styrene terpolymer.
Clause 9: The stopper of any of clauses 1 to 8, wherein the stopper body has an Aᵣₑₐₗ/Aₒ in the range of about 0.42 to about 0.70 on at least one sealing rib.
Clause 10: The stopper of any of clauses 1 to 9, wherein the stopper body further comprises at least two sealing ribs formed thereon.
Clause 11: The stopper of any of clauses 1 to 10, wherein at least one of the sealing ribs is laminated with at least one lamination layer.
Clause 12: The stopper of any of clauses 1 to 10, wherein all of the sealing ribs are laminated with at least one lamination layer.
Clause 13: The stopper of any of clauses 1 to 10, wherein all of the sealing ribs and the stopper body are laminated with at least one lamination layer.
Clause 14: The stopper of any of clauses 11 to 13, wherein the at least one lamination layer is formed from at least one compound selected form densified expanded polytetrafluoroethylene (ePTFE), polytetrafluoroethylene (PTFE), fluorinated ethylene propylene (FEP), a polyethylene such as a UHMWPE, polypropylene, polyvinylidene fluoride, polyvinylfluoride, perfluoropropylevinylether, a perfluoroalkoxy polymer, and copolymers and combinations thereof.
Clause 15: The stopper of clause 14, wherein the at least one lamination layer is formed from at least one layer of polytetrafluoroethylene (PTFE) or UHMWPE.
Clause 16: The stopper of any of clauses 1 to 15, wherein the surface roughness of the polymeric material that is used to form the stopper of the present invention is in a range of about 0.0002 microns to about 4 microns as measured at 50X via optical interferometry.
Clause 17: A syringe comprising: a syringe barrel; a plunger, wherein the plunger is designed to fit within the syringe barrel, wherein the syringe barrel has a compression ratio (CR) of about 1 percent to about 7.4 percent where CR is defined as the initial value before assembly such that CR = 100% x (R_{free stopper OD} - R_{barrel ID}) / R_{barrel ID}; and a stopper according to any of clauses 1 to 16, wherein the stopper is adapted for attachment to one end of the plunger so as act as a seal for the syringe barrel.
Clause 18: A sealing device comprising: a sealing body formed from a polymeric material; and at least one sealing structure and/or or sealing rib formed on at least one portion of the sealing body, wherein the polymeric material is selected from one or more polyolefins, one or more polyamides, one or more polyesters, one or more polystyrenes, one or more polyurethanes, one or more polycarbonates, one or more acrylonitrile-butadiene-styrenes, one or more fluoropolymers, one or more polyacrylates, one or more elastomeric rubbers, one or more polyisoprenes, one or more polyisobutylene, one or more polybutadienes, one or more chloroprene rubbers, one or more butyl rubbers, one or more halogenated butyl rubbers, one or more styrene-butadiene rubbers, one or more nitrile rubbers, one or more hydrogenated nitrile rubbers, natural rubber, one or more ethylene propylene rubbers, one or more ethylene propylene diene rubbers, or blends of any two or more thereof where the polymeric material, or the blend of polymeric materials, has a Young's modulus in a range of about 5 MPa to about 10 MPa and a Shore A hardness in a range of about 50 to about 85.

### BRIEF DESCRIPTION OF THE DRAWINGS

Figure 1 is perspective view of an exemplary syringe with which embodiments of the disclosure may be implemented;
Figure 2 is a perspective view of the syringe of Figure 1 showing a needle shield covering a distal end of the syringe;
Figure 3 is a perspective view of a stopper according to a first embodiment of the present invention;
Figure 4 is a cross-sectional view of one stopper design according to one embodiment of the present invention where the stopper contains an Acme thread therein;
Figure 5 is a cross-sectional view of one stopper design according to another embodiment of the present invention where the stopper contains a snap-in plunger rod connector therein;
Figure 6 is a cross-sectional view of one stopper design according to still another embodiment of the present invention where the stopper contains a buttress tread therein;
Figure 7 is a cross-sectional view of one stopper design according to still another embodiment of the present invention where the stopper contains a buttress tread therein; and
Figure 8 is a cross-sectional view of one stopper design according to still another embodiment of the present invention where the stopper contains a buttress tread therein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

As noted above, the invention relates, in general, to various methods to manufacture and/or design stoppers, in particular laminated stoppers for medical applications, as well as various polymer blends and design considerations to be considered when manufacturing such stoppers. In one embodiment, the present invention relates to a syringe stopper formed from a material having an increased rubber modulus and/or a compression ratio, as defined below, that yields a partially laminated stopper with only one or more desired sealing services being laminated (e.g. a stopper with, for example, three ribs but only one or two thereof being a laminated sealing rib) or a fully laminated stopper with all sealing services being laminated (e.g. a stopper with, for example, three ribs where all three ribs are laminated sealing ribs) sealing surfaces, stopper with suitable contact pressures (Aᵣₑₐₗ/Aₒ is greater than 0.42) so as to realize total sealing in a wide variety of applications.

Initially, a discussion of one set of exemplary stopper designs will be undertaken. However, it is to be understood that the following stopper design is only representative and the present invention is not limited to solely the designs disclosed herein. Rather, any stopper design where total sealing is desired can be utilized in conjunction with the present invention. Specifically, the type of plunger rod could have either Buttress or ACME type of thread and different levels of stopper-plunger rod (PR) mechanical interference are possible. Since the stopper functional use should guarantee CCI even when not coupled to a PR for shelf life, the level of plunger rod-stopper interference when assembled is not a critical parameter. However, it is understood that high contact pressures can be delivered by increasing PR-stopper interference in cases where shelf life occurs in the stopper-PR assembled state. It should be noted that due to the high Poisson's ratio of low modulus elastomeric materials (v greater than 0.4) the use of high PR-stopper interference or compression ratios with low modulus rubbers is a non-effective way to achieve percolation threshold as manifested on previous art.

For purposes of the description hereinafter, the terms "upper", "lower", "right", "left", "vertical", "horizontal", "top", "bottom", "lateral", "longitudinal", and derivatives thereof, shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

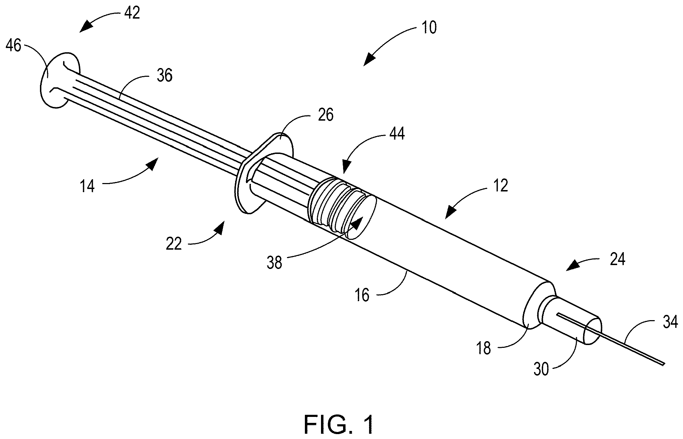

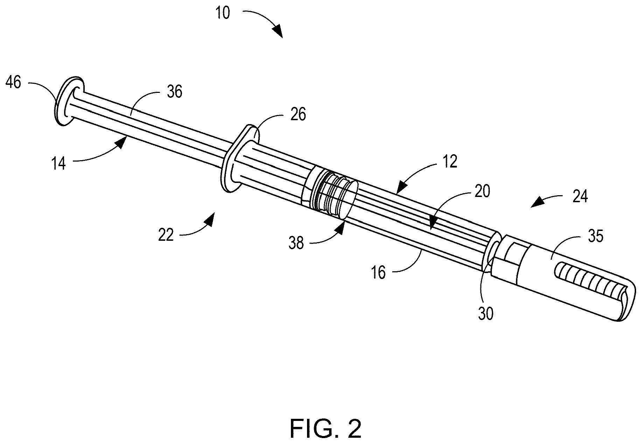

Referring to Figures 1 and 2, shown is a non-limiting embodiment of a syringe 10 with which aspects or embodiments of the disclosure may be implemented. According to some aspects of the disclosure, the syringe 10 may be provided as a prefilled syringe, which provides the convenience of rapidly delivering the liquid therein to a patient without the need to first aspirate the medication from another container and meter its volume.

As shown in to Figures 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed from a suitable glass or plastic material and includes a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a hub 30 which narrows with respect to the cylindrical outer wall 16 and extends out distally therefrom. The hub portion 30 is formed as a partially hollow member that defines a channel therethrough in fluid communication with the chamber 20. A needle 34 is attached to the hub portion 30 within the channel, such as by being glued or otherwise secured to the hub portion 30. According to some aspects of the disclosure, syringe 10 may further include a cover 35 that couples to the hub portion 30 of syringe barrel 12 to protect the needle 34.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. A thumb press 46 is positioned at the plunger proximal end 42 that may be engaged by a thumb (or other finger) of the user to apply a distally directed force to the plunger assembly 14 to move the plunger 36 with respect to the syringe barrel 12. The plunger distal end 44 is configured to mate with the stopper 38. In other embodiments, the plunger distal end 44 may be a threaded end that mates with the stopper 38.

The stopper 38 of plunger assembly 14 is positioned at the plunger distal end 44 so as to be movable along with the plunger 36 within the chamber 20 of syringe barrel 12. That is, as a distally-directed force is applied to the plunger rod 36 (such as to thumb press 46), both the plunger rod 36 and the stopper 38 are caused to move distally through syringe barrel 12, with the stopper 38 configured to maintain a seal with an inner surface of the syringe barrel 12 as it is moved therethrough.

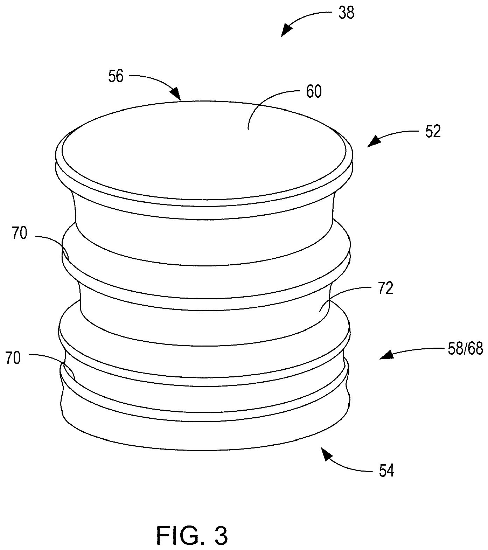

Referring now to Figure 3, and with continued reference to Figures 1 and 2, the structure of an exemplary stopper 38 that may be included in syringe 10 is shown in greater detail, according to a non-limiting aspect or embodiment of the disclosure. The stopper 38 is defined by a main body 52 that includes a proximal end 54 and a distal end 56. The proximal end 54 may be configured as an open proximal end that engages with the distal end of plunger 36, while the distal end 56 is configured as a closed distal end configured to engage with the barrel 12 of syringe 10. The closed distal end 56 of the main body 52 includes a generally cylindrical portion 58 and a roof portion 60. In some embodiments, the roof portion 60 is configured as a flat surface to provide the closed distal end 56, but it is recognized that the roof portion 60 may instead be configured as a conical or domed surface, as other non-limiting examples.

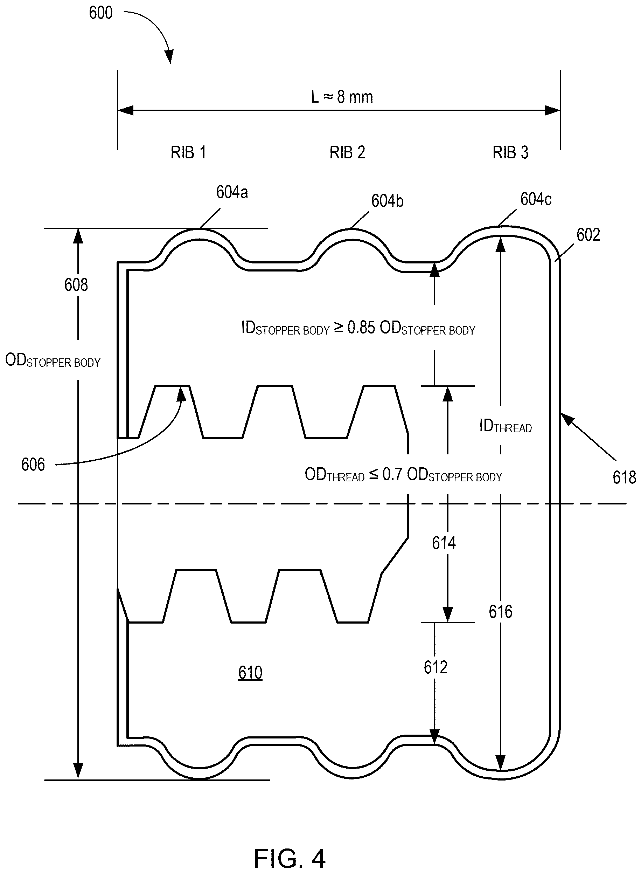

As known in the art, the main body 52 of the stopper 38 is partially hollow and is sized and configured to receive the distal end 44 of plunger 36 therein. The main body 52 of the stopper 38 defines an inner cavity (visible in Figures 4-8) that, in some embodiments, may include a threaded inner surface that is configured to receive and mate with a threaded distal end 44 of plunger 36. In other embodiments, the inner cavity may define an inner contoured surface having a notch therein that is configured to receive a flanged extension member of plunger 36.

The outer surface 68 of the cylindrical portion 58 of the main body 52 includes one or more ribs thereon that extend circumferentially around the entire outer surface 68 of the cylindrical portion 58. In each of the illustrated embodiments, a plurality of ribs 70 are included on the stopper 38, but it is recognized that other embodiments could include only a single rib 70. The ribs 70 may be formed integrally with the main body 52, such as via a molding process. The plurality of ribs 70 may include two ribs 70, with a first or distal rib positioned toward the distal end 56 of the main body 52 and a second or proximal rib positioned toward the proximal end 54 of the main body 52, or may include three or more ribs 70 spaced apart along the cylindrical portion 58 - with an inter-rib region 72 provided between each adjacent pair of ribs. The inter-rib region 72 of cylindrical portion 58 may have a generally flat outer surface 68 or a curved outer surface 68.

The stopper 38 may be made from a material that is different from the material of the plunger 36 and that is capable of forming a tight seal with the syringe barrel 12 as it is advanced therethrough. In some embodiments, the stopper 38 is desirably manufactured from any suitable polymeric material with a Young's modulus (also known as the tensile modulus, elastic modulus or traction modulus) in a range of about 5 MPa to about 10 MPa, or from about 5.25 MPa to about 9.75 MPa, or from about 5.5 MPa to about 9.5 MPa, or from about 5.75 MPa to about 9.25 MPa, or from about 6 MPa to about 9 MPa, or from about 6.25 MPa to about 8.75 MPa, or from about 6.5 MPa to about 8.5 MPa, or from about 6.75 MPa to about 8.25 MPa, or from about 7 MPa to about 8 MPa, or from about 7.25 MPa to about 7.75 MPa, or even about 7.5 MPa. In another embodiment, stopper 38 is desirably manufactured from any suitable polymeric material with a Young's modulus (also known as the tensile modulus, elastic modulus or traction modulus) in a range of about 5 MPa to about 10 MPa, or from about 5.1 MPa to about 9.9 MPa, or from about 5.2 MPa to about 9.8 MPa, or from about 5.3 MPa to about 9.7 MPa, or from about 5.4 MPa to about 9.6 MPa, or from about 5.5 MPa to about 9.5 MPa, or from about 5.6 MPa to about 9.4 MPa, or from about 5.7 MPa to about 9.3 MPa, or from about 5.8 MPa to about 9.2 MPa, or from about 5.9 MPa to about 9.1 MPa, or from about 6 MPa to about 9 MPa, or from about 6.1 MPa to about 8.9 MPa, or from about 6.2 MPa to about 8.8 MPa, or from about 6.3 MPa to about 8.7 MPa, or from about 6.4 MPa to about 8.6 MPa, or from about 6.5 MPa to about 8.5 MPa, or from about 6.6 MPa to about 8.4 MPa, or from about 6.7 MPa to about 8.3 MPa, or from about 6.8 MPa to about 8.2 MPa, or from about 6.9 MPa to about 8.1 MPa, or from about 7 MPa to about 8 MPa, or from about 7.1 MPa to about 7.9 MPa, or from about 7.2 MPa to about 7.8 MPa, or from about 7.3 MPa to about 7.7 MPa, or from about 7.4 MPa to about 7.6 MPa, or even about 7.5 MPa. Here, as well as elsewhere in the specification and claims, individual numerical values can be combined to form additional, or even new/non-disclosed, numerical ranges.

The desired Shore A hardness of the afore-mentioned suitable polymer materials for stopper 38 of the present invention is between about 50 to about 85, or from about 55 to about 80, or from about 60 to about 75, or from about 65 to about 70, or even about 67.5. In another embodiment, stopper 38 is desirably manufactured from any suitable polymeric material with a Shore A hardness of about 50 to about 85, or from about 52 to about 83, or from about 54 to about 81, or from about 56 to about 79, or from about 58 to about 77, or from about 60 to about 75, or from about 62 to about 73, or from about 64 to about 71, or from about 66 to about 69, or even about 67.5. Here, as well as elsewhere in the specification and claims, individual numerical values can be combined to form additional, or even new/non-disclosed, numerical ranges.

Suitable stopper polymeric materials include, but are not limited to, one or more polyolefins (e.g., PE, PP, and their copolymers), one or more polyamides (e.g., nylons), one or more polyesters (e.g., PET), one or more polystyrenes, one or more polyurethanes, one or more polycarbonates, one or more acrylonitrile-butadiene-styrenes, one or more fluoropolymers, one or more polyacrylates, one or more elastomeric rubbers, or blends of any two or more thereof that meet at least one of the Young's modulus and/or Shore A properties defined above. In another embodiment, suitable stopper materials include, but are not limited to, polymeric materials selected from one or more polyisoprenes (including, but not limited to, natural rubber), one or more polyisobutylenes, one or more synthetic polyisoprenes, one or more polybutadienes (commonly referred to collectively as butadiene rubbers), one or more chloroprene rubbers (e.g., polychloroprene, neoprene, etc.), one or more butyl rubbers (i.e., copolymers of isobutene and isoprene), one or more halogenated butyl rubbers (e.g., chloro butyl rubber or bromo butyl rubber), one or more styrene-butadiene rubbers (i.e., copolymers of styrene and butadiene), one or more nitrile rubbers (i.e., copolymers of butadiene and acrylonitrile), one or more hydrogenated nitrile rubbers, natural rubber, one or more ethylene propylene rubbers, one or more ethylene propylene diene rubbers, or blends of any two or more thereof. In another embodiment, suitable stopper polymeric materials include, but are not limited to, one or more butadiene rubbers, one or more halogenated butyl rubbers (e.g., chloro butyl rubber or bromo butyl rubber), or blends of any two or more thereof.

In another embodiment, stopper 38 is desirably manufactured from any suitable polymeric material with either a Young's modulus and/or a Shore A hardness in one of the ranges noted above in combination with a high Mooney viscosity, where the base polymer material's Mooney viscosity is determined before curing. A high Mooney rubber is defined as rubber having Mooney viscosity (as defined in ASTM D1646-19a) greater than about 40, or greater than about 45, or greater than about 50, or greater than about 55, or greater than about 60, or greater than about 65, or even greater than about 70. Here, as well as elsewhere in the specification and claims, individual numerical values can be combined to form additional, or even new/non-disclosed, numerical ranges.

In another embodiment, stopper 38 is desirably manufactured to have a compression ratio (CR) with a syringe barrel's inner diameter (ID) between about 1 percent and about 7.4 percent, or from about 1.2 percent to about 7.2 percent, or from about 1.4 percent to about 7 percent, or from about 1.6 percent to about 6.8 percent, or from about 1.8 percent to about 6.6 percent, or from about 2 percent to about 6.4 percent, or from about 2.2 percent to about 6.2 percent, or from about 2.4 percent to about 6 percent, or from about 2.6 percent to about 5.8 percent, or from about 2.8 percent to about 5.6 percent, or from about 3 percent to about 5.4 percent, or from about 3.2 percent to about 5.2 percent, or from about 3.4 percent to about 5 percent, or from about 3.6 percent to about 4.8 percent, or from about 3.8 percent to about 4.6 percent, or from about 4 percent to about 4.4 percent, or even about 4.2 percent, according to any of the embodiments disclosed herein, wherein the stopper is adapted for attachment to one end of the plunger so as act as a seal for the syringe barrel. As noted above, the compression ratio (CR) is defined as the initial value before assembly of CR = 100% x (R_{free stopper OD} - R_{barrel ID}) / R_{barrel ID}. Here, as well as elsewhere in the specification and claims, individual numerical values can be combined to form additional, or even new/non-disclosed, numerical ranges.

Suitable high Mooney viscosity rubbers include, but are not limited to, one or more polybutadiene rubbers, one or more polyisobutylene, one or more polyisoprene rubbers, natural rubber, one or more ethylene propylene rubbers, one or more ethylene propylene diene rubbers, one or more styrene-butadiene rubbers, or blends of any two or more thereof.

In one embodiment, stopper 38 is manufactured from a polymeric material with a significantly lower coefficient of friction and contact area/pressure relative to a conventional rubber stopper. Accordingly, in this embodiment, stopper 38 can be used with a syringe 10 without silicone oil or other lubricants. In addition, the syringe barrel 12 may be made of the same material, a similar material, or even a completely different material from that used to form stopper 38.

With reference now to Figures 4-8, additional stopper body designs are shown in accordance with embodiments of the present invention.

Turning to Figure 4, Figure 4 discloses a stopper 600 having a laminated surface 602 thereon. As can be seen from Figure 4, stopper 600 has three ribs 604a, 604b, and 604c. Although stopper 600 has been shown with three ribs, ribs 604a, 604b, and 604c, the present invention is not limited thereto. Rather, as noted herein, any suitable number of ribs can be formed in stopper 600 as desired by the design parameters under consideration for stopper 600.

Continuing on further, stopper 600 further has a threaded portion 606, which in a non-limiting instance, can be formed in the shape of an Acme thread structure 606. As disclosed therein, stopper 600 can be any suitable size as determined by its length and width. In one non-limiting embodiment, stopper 600 is about 8 mm in length and has a width 608 (defined as OD_{STOPPER BODY}) at its widest point that is determined, per one non-limiting instance, by the compression ratio necessary to achieve the desired design parameters of the present invention. In the case where stopper 600 is about 8 mm in length, the radii of ribs 604a, 604b, and 604c are, in one non-limiting instance, about 5 mm or less. In another instance, each of ribs 604a, 604b, and 604c can independently each have their own rib radius so long as each different rib radius is about 5 mm or less. The body 610 of stopper 600 can be formed from any suitable polymeric material disclosed herein.

Continuing on with regard to stopper 600, in one non-limiting example, as is illustrated in Figure 4, the inner diameter 612 (ID_{STOPPER BODY}) of stopper 600 is designed to be greater than or equal to 85 percent (i.e., 0.85) of the outer diameter 608 (OD_{STOPPER BODY}) of stopper 600. That is, ID_{STOPPER BODY} ≥ 0.85 OD_{STOPPER BODY}. Additionally, in one non-limiting embodiment, the outer diameter 614 (OD_{THREAD}) of thread 606 is formed such that the outer diameter 614 (OD_{THREAD}) is less than or equal to 70 percent (i.e., 0.70) of the outer diameter 608 (OD_{STOPPER BODY}) of stopper 600. That is, OD_{THREAD} ≤ 0.7 OD_{STOPPER BODY}.

In one embodiment, stopper 600 has a flat front surface 618, but as would be appreciated by those of skill in the art could alternatively have any desired geometric design at the front surface thereof. As such, stopper 600 of Figure 4 has, in one non-limiting embodiment, three ribs, an Acme thread, a flat front, and is fully laminated. However, as is discussed herein, modifications to the design of stopper 600 are fully within the scope of the present invention and can include one or more exemplary non-limiting modifications such as a different number of ribs, one or more laminated ribs, one or more non-laminated ribs, a non-flat front surface, etc.

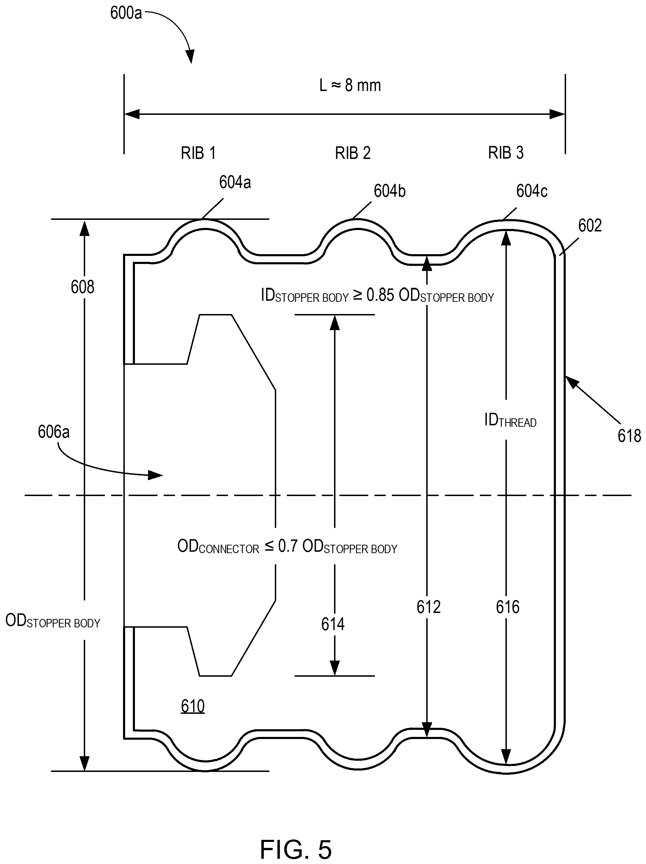

Turning to Figure 5, this figure represents a stopper design 600a where Acme thread structure 606 of stopper 600 has been replaced by a snap-in plunger rod connector 606a. Regarding the various geometric relationships of the stopper design of stopper 600a, these relationships are similar to that of stopper 600 where the inner diameter 612 (ID_{STOPPER BODY}) of stopper 600a is designed to be greater than or equal to 85 percent (i.e., 0.85) of the outer diameter 608 (OD_{STOPPER BODY}) of stopper 600a. That is, ID_{STOPPER BODY} ≥ 0.85 OD_{STOPPER BODY}. Additionally, in one non-limiting embodiment, the outer diameter 614 (OD_{CONNECTOR}) of connector 606a is formed such that the outer diameter 614 (OD_{CONNECTOR}) is less than or equal to 70 percent (i.e., 0.70) of the outer diameter 608 (OD_{STOPPER BODY}) of stopper 600a. That is, OD_{CONNECTOR} ≤ 0.7 OD_{STOPPER} BODY.

Although stopper 600a has been shown with three ribs, ribs 604a, 604b, and 604c, the present invention is not limited thereto. Rather, as noted herein, any suitable number of ribs can be formed in stopper 600a as desired by the design parameters under consideration for stopper 600a. Additionally, the body 610 of stopper 600a can be formed from any suitable polymeric material disclosed herein.

In one embodiment, stopper 600a has a flat front surface 618, but as would be appreciated by those of skill in the art could alternatively have any desired geometric design at the front surface thereof. As such, stopper 600a of Figure 5 has, in one non-limiting embodiment, three ribs, a snap-in plunger rod connector, a flat front, and is fully laminated. However, as is discussed herein, modifications to the design of stopper 600a are fully within the scope of the present invention and can include one or more exemplary non-limiting modifications such as a different number of ribs, one or more laminated ribs, one or more non-laminated ribs, a non-flat front surface, etc. Additionally, in one non-limiting embodiment, stopper 600a is also about 8 mm in length and has a width 608 (defined as OD_{STOPPER BODY}) at its widest point that is determined, per one non-limiting instance, by the compression ratio necessary to achieve the desired design parameters of the present invention.

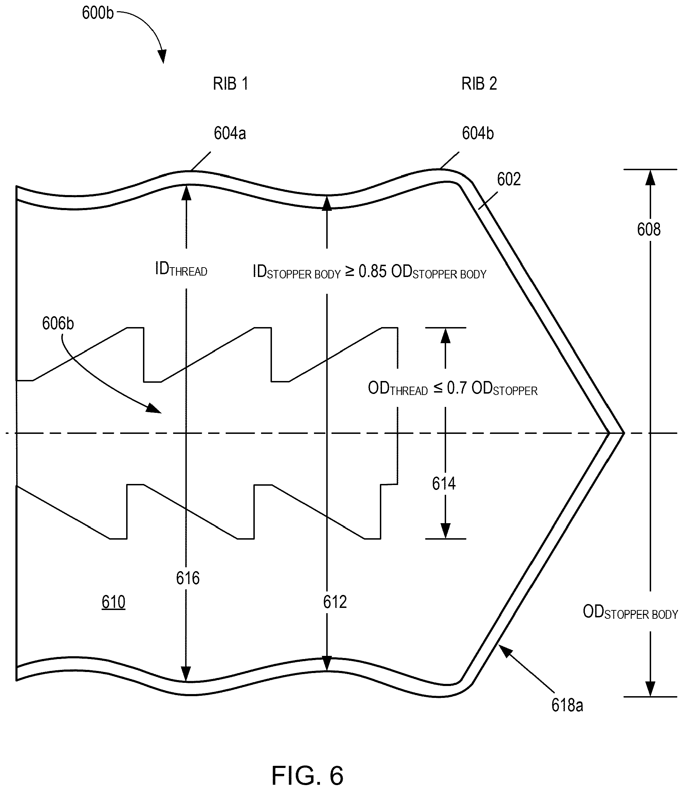

Turning to Figure 6, this figure represents a stopper design 600b where Acme thread structure 606 has been replaced by a buttress thread structure 606b and where stopper 600b only has two ribs 604a and 604b. Regarding the various geometric relationships of the stopper design of stopper 600b, these relationships are similar to that of stopper 600 where the inner diameter 612 (ID_{STOPPER BODY}) of stopper 600b is designed to be greater than or equal to 85 percent (i.e., 0.85) of the outer diameter 608 (OD_{STOPPER BODY}) of stopper 600b. That is, ID_{STOPPER BODY} ≥ 0.85 OD_{STOPPER BODY}. Additionally, in one non-limiting embodiment, the outer diameter 614 (OD_{THREAD}) of thread 606b is formed such that the outer diameter 614 (OD_{THREAD}) is less than or equal to 70 percent (i.e., 0.70) of the outer diameter 608 (OD_{STOPPER BODY}) of stopper 600b. That is, OD_{THREADR} ≤ 0.7 OD_{STOPPER BODY}.

Although stopper 600b has been shown with two ribs, ribs 604a and 604b, the present invention is not limited thereto. Rather, as noted herein, any suitable number of ribs can be formed in stopper 600b as desired by the design parameters under consideration for stopper 600b. Additionally, the body 610 of stopper 600b can be formed from any suitable polymeric material disclosed herein.

In one embodiment, stopper 600b has a conically-shaped front surface 618a, but as would be appreciated by those of skill in the art could alternatively have any desired geometric design at the front surface thereof. As such, stopper 600b of Figure 6 has, in one non-limiting embodiment, two ribs, a buttress thread, a conically-shaped front, and is fully laminated. However, as is discussed herein, modifications to the design of stopper 600b are fully within the scope of the present invention and can include one or more exemplary non-limiting modifications such as a different number of ribs, one or more laminated ribs, one or more non-laminated ribs, a non-flat front surface, etc. Additionally, in one non-limiting embodiment, stopper 600b has a width 608 (defined as OD_{STOPPER BODY}) at its widest point that is determined, per one non-limiting instance, by the compression ratio necessary to achieve the desired design parameters of the present invention.

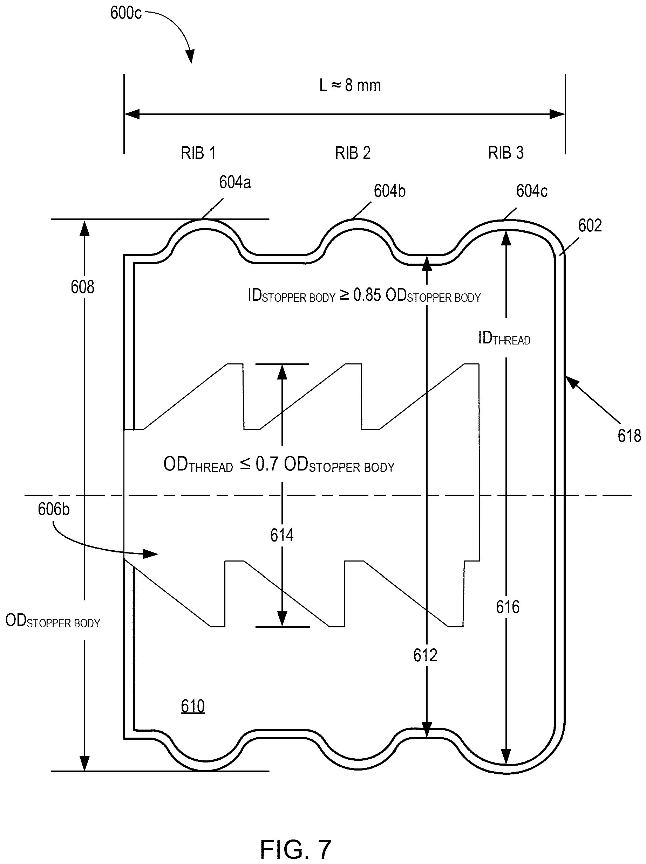

Turning to Figure 7, this figure represents a stopper design 600c where Acme thread structure 606 of stopper 600 has been replaced by a buttress thread structure 606b. Regarding the various geometric relationships of the stopper design of stopper 600c, these relationships are similar to that of stopper 600 where the inner diameter 612 (ID_{STOPPER BODY}) of stopper 600c is designed to be greater than or equal to 85 percent (i.e., 0.85) of the outer diameter 608 (OD_{STOPPER BODY}) of stopper 600c. That is, ID_{STOPPER BODY} ≥ 0.85 OD_{STOPPER BODY}. Additionally, in one non-limiting embodiment, the outer diameter 614 (OD_{THREAD}) of thread 606b is formed such that the outer diameter 614 (OD_{THREAD}) is less than or equal to 70 percent (i.e., 0.70) of the outer diameter 608 (OD_{STOPPER BODY}) of stopper 600c. That is, OD_{THREAD} ≤ 0.7 OD_{STOPPER BODY}.

Although stopper 600c of Figure 7 is illustrated having three ribs 604a, 604b, and 604c, the present invention is not limited thereto. Rather, as noted herein, any suitable number of ribs can be formed in stopper 600c as desired by the design parameters under consideration for stopper 600c. Additionally, the body 610 of stopper 600c can be formed from any suitable polymeric material disclosed herein.

In one embodiment, stopper 600c has a flat front surface 618, but as would be appreciated by those of skill in the art could alternatively have any desired geometric design at the front surface thereof. As such, stopper 600c of Figure 7 has, in one non-limiting embodiment, three ribs, a buttress thread, a flat front, and is fully laminated. However, as is discussed herein, modifications to the design of stopper 600c are fully within the scope of the present invention and can include one or more exemplary non-limiting modifications such as a different number of ribs, one or more laminated ribs, one or more non-laminated ribs, a non-flat front surface, etc. Additionally, in one non-limiting embodiment, stopper 600c has a width 608 (defined as OD_{STOPPER BODY}) at its widest point that is determined, per one non-limiting instance, by the compression ratio necessary to achieve the desired design parameters of the present invention.

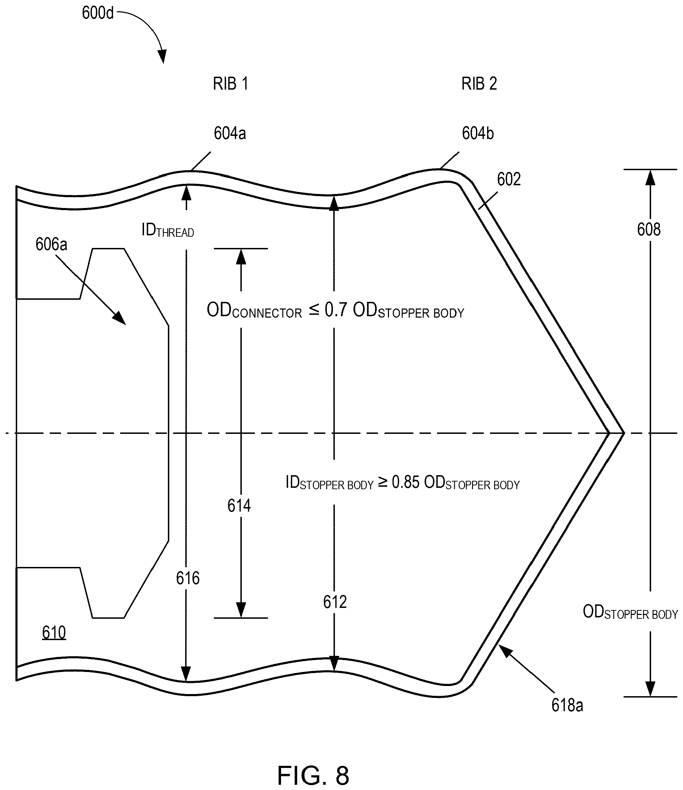

Turning to Figure 8, this figure represents a stopper design 600d where buttress thread structure 606b has been replaced by a snap-in plunger rod connector 606a and where stopper 600d only has two ribs 604a and 604b similar to stopper 600b. Regarding the various geometric relationships of the stopper design of stopper 600d, these relationships are similar to that of stopper 600b where the inner diameter 612 (ID_{STOPPER BODY}) of stopper 600d is designed to be greater than or equal to 85 percent (i.e., 0.85) of the outer diameter 608 (OD_{STOPPER BODY}) of stopper 600d. That is, ID_{STOPPER BODY} ≥ 0.85 OD_{STOPPER BODY}. Additionally, in one non-limiting embodiment, the outer diameter 614 (OD_{CONNECTOR}) of connector 606a is formed such that the outer diameter 614 (OD_{CONNECTOR}) is less than or equal to 70 percent (i.e., 0.70) of the outer diameter 608 (OD_{STOPPER BODY}) of stopper 600b. That is, OD_{CONNECTOR} ≤ 0.7 OD_{STOPPER BODY}.

Although stopper 600d has been shown with two ribs, ribs 604a and 604b, the present invention is not limited thereto. Rather, as noted herein, any suitable number of ribs can be formed in stopper 600d as desired by the design parameters under consideration for stopper 600b. Additionally, the body 610 of stopper 600d can be formed from any suitable polymeric material disclosed herein.

In one embodiment, stopper 600d has a conically-shaped front surface 618a, but as would be appreciated by those of skill in the art could alternatively have any desired geometric design at the front surface thereof. As such, stopper 600d of Figure 8 has, in one non-limiting embodiment, two ribs, a snap-in plunger rod connector, a conically-shaped front, and is fully laminated. However, as is discussed herein, modifications to the design of stopper 600d are fully within the scope of the present invention and can include one or more exemplary non-limiting modifications such as a different number of ribs, one or more laminated ribs, one or more non-laminated ribs, a non-flat front surface, etc. Additionally, in one non-limiting embodiment, stopper 600d has a width 608 (defined as OD_{STOPPER BODY}) at its widest point that is determined, per one non-limiting instance, by the compression ratio necessary to achieve the desired design parameters of the present invention.

In one embodiment, due to the polymeric material that is used to form the stopper of the present invention (or any of the disclosed alternative stopper designs incorporated herein by reference), suitable sealing is accomplished by the contact pressure achieved by any one or more of a stopper in accordance with the present invention where such a stopper is formed from any one or more of the polymeric materials having one or more of the properties disclosed herein. That is, the suitable sealing properties of the stoppers of the present invention are achieved due to one or more of stopper geometry, the Young's modulus of the polymeric material used to form such a stopper, the indentation modulus of the laminate sealing surface, and/or the roughness of both the surface of the stopper and/or the inner surface of the barrel (or other container) that the stopper is designed to seal.

In one embodiment, the surface roughness of the glass, polymer, metal or another type of material that forms the surface to be sealed (such as a vial, syringe, or other type of container) by the stopper of the present invention is independently in a range of about 0.0001 microns to about 6 microns, or from about 0.0002 microns to about 5.9 microns, or from about 0.0003 microns to about 5.8 microns, or from about 0.0004 microns to about 5.7 microns, or from about 0.0005 microns to about 5.6 microns, or from about 0.0006 microns to about 5.5 microns, or from about 0.0007 microns to about 5.4 microns, or from about 0.0008 microns to about 5.3 microns, or from about 0.0009 microns to about 5.2 microns, or from about 0.001 microns to about 5.1 microns, or from about 0.0011 microns to about 5 microns, or from about 0.0012 microns to about 4.9 microns, or from about 0.0013 microns to about 4.8 microns, or from about 0.0014 microns to about 4.7 microns, or from about 0.0015 microns to about 4.6 microns, or from about 0.0016 microns to about 4.5 microns, or from about 0.0017 microns to about 4.4 microns, or from about 0.0018 microns to about 4.3 microns, or from about 0.0019 microns to about 4.2 microns, or from about 0.002 microns to about 4.1 microns, or from about 0.0021 microns to about 4 microns, or from about 0.0022 microns to about 3.9 microns, or from about 0.0023 microns to about 3.8 microns, or from about 0.0024 microns to about 3.7 microns, or from about 0.0025 microns to about 3.6 microns, or from about 0.0026 microns to about 3.5 microns, or from about 0.0027 microns to about 3.4 microns, or from about 0.0028 microns to about 3.3 microns, or from about 0.0029 microns to about 3.2 microns, or from about 0.003 microns to about 3.1 microns, or from about 0.0031 microns to about 3 microns, or from about 0.0032 microns to about 2.9 microns, or from about 0.0033 microns to about 2.8 microns, or from about 0.0034 microns to about 2.7 microns, or from about 0.0035 microns to about 2.6 microns, or from about 0.0036 microns to about 2.5 microns, or from about 0.0037 microns to about 2.4 microns, or from about 0.0038 microns to about 2.3 microns, or from about 0.0039 microns to about 2.2 microns, or from about 0.004 microns to about 2.1 microns, or from about 0.0041 microns to about 2 microns, or from about 0.0042 microns to about 1.9 microns, or from about 0.0043 microns to about 1.8 microns, or from about 0.0044 microns to about 1.7 microns, or from about 0.0045 microns to about 1.6 microns, or from about 0.0046 microns to about 1.5 microns, or from about 0.0047 microns to about 1.4 microns, or from about 0.0048 microns to about 1.3 microns, or from about 0.0049 microns to about 1.2 microns, or from about 0.005 microns to about 1.1 microns, or from about 0.0051 microns to about 1 micron, or from about 0.0052 microns to about 0.9 microns, or from about 0.0053 microns to about 0.8 microns, or from about 0.0054 microns to about 0.7 microns, or from about 0.0055 microns to about 0.6 microns, or from about 0.0056 microns to about 0.5 microns, or from about 0.0057 microns to about 0.4 microns, or from about 0.0058 microns to about 0.3 microns, or from about 0.0059 microns to about 0.2 microns, or from about 0.006 microns to about 0.1 microns, or from about 0.0065 microns to about 0.095 microns, or from about 0.007 microns to about 0.09 microns, or from about 0.0075 microns to about 0.085 microns, or from about 0.008 microns to about 0.08 microns, or from about 0.0085 microns to about 0.075 microns, or from about 0.009 microns to about 0.07 microns, or from about 0.0095 microns to about 0.065 microns, or from about 0.01 microns to about 0.06 microns, or from about 0.015 microns to about 0.055 microns, or from about 0.02 microns to about 0.05 microns, or from about 0.025 microns to about 0.045 microns, or from about 0.03 microns to about 0.04 microns, or even 0.035 microns as measured at 50X via optical interferometry. Here, as well as elsewhere in the specification and claims, individual numerical values can be combined to form additional, or even new/non-disclosed, numerical ranges.

In one embodiment, the surface roughness of the polymeric material that is used to form the stopper of the present invention is independently in a range of about 0.0001 microns to about 6 microns, or from about 0.0002 microns to about 5.9 microns, or from about 0.0003 microns to about 5.8 microns, or from about 0.0004 microns to about 5.7 microns, or from about 0.0005 microns to about 5.6 microns, or from about 0.0006 microns to about 5.5 microns, or from about 0.0007 microns to about 5.4 microns, or from about 0.0008 microns to about 5.3 microns, or from about 0.0009 microns to about 5.2 microns, or from about 0.001 microns to about 5.1 microns, or from about 0.0011 microns to about 5 microns, or from about 0.0012 microns to about 4.9 microns, or from about 0.0013 microns to about 4.8 microns, or from about 0.0014 microns to about 4.7 microns, or from about 0.0015 microns to about 4.6 microns, or from about 0.0016 microns to about 4.5 microns, or from about 0.0017 microns to about 4.4 microns, or from about 0.0018 microns to about 4.3 microns, or from about 0.0019 microns to about 4.2 microns, or from about 0.002 microns to about 4.1 microns, or from about 0.0021 microns to about 4 microns, or from about 0.0022 microns to about 3.9 microns, or from about 0.0023 microns to about 3.8 microns, or from about 0.0024 microns to about 3.7 microns, or from about 0.0025 microns to about 3.6 microns, or from about 0.0026 microns to about 3.5 microns, or from about 0.0027 microns to about 3.4 microns, or from about 0.0028 microns to about 3.3 microns, or from about 0.0029 microns to about 3.2 microns, or from about 0.003 microns to about 3.1 microns, or from about 0.0031 microns to about 3 microns, or from about 0.0032 microns to about 2.9 microns, or from about 0.0033 microns to about 2.8 microns, or from about 0.0034 microns to about 2.7 microns, or from about 0.0035 microns to about 2.6 microns, or from about 0.0036 microns to about 2.5 microns, or from about 0.0037 microns to about 2.4 microns, or from about 0.0038 microns to about 2.3 microns, or from about 0.0039 microns to about 2.2 microns, or from about 0.004 microns to about 2.1 microns, or from about 0.0041 microns to about 2 microns, or from about 0.0042 microns to about 1.9 microns, or from about 0.0043 microns to about 1.8 microns, or from about 0.0044 microns to about 1.7 microns, or from about 0.0045 microns to about 1.6 microns, or from about 0.0046 microns to about 1.5 microns, or from about 0.0047 microns to about 1.4 microns, or from about 0.0048 microns to about 1.3 microns, or from about 0.0049 microns to about 1.2 microns, or from about 0.005 microns to about 1.1 microns, or from about 0.0051 microns to about 1 micron, or from about 0.0052 microns to about 0.9 microns, or from about 0.0053 microns to about 0.8 microns, or from about 0.0054 microns to about 0.7 microns, or from about 0.0055 microns to about 0.6 microns, or from about 0.0056 microns to about 0.5 microns, or from about 0.0057 microns to about 0.4 microns, or from about 0.0058 microns to about 0.3 microns, or from about 0.0059 microns to about 0.2 microns, or from about 0.006 microns to about 0.1 microns, or from about 0.0065 microns to about 0.095 microns, or from about 0.007 microns to about 0.09 microns, or from about 0.0075 microns to about 0.085 microns, or from about 0.008 microns to about 0.08 microns, or from about 0.0085 microns to about 0.075 microns, or from about 0.009 microns to about 0.07 microns, or from about 0.0095 microns to about 0.065 microns, or from about 0.01 microns to about 0.06 microns, or from about 0.015 microns to about 0.055 microns, or from about 0.02 microns to about 0.05 microns, or from about 0.025 microns to about 0.045 microns, or from about 0.03 microns to about 0.04 microns, or even 0.035 microns as measured at 50X via optical interferometry. Here, as well as elsewhere in the specification and claims, individual numerical values can be combined to form additional, or even new/non-disclosed, numerical ranges.

In still another embodiment, if the surface to be sealed is partially, or fully, laminated the surface roughness of the laminated film, or the top most laminated film in the case where two or more films are used to provide an overall laminated coating, is independently in a range of about 0.0001 microns to about 12 microns, or from about 0.0002 microns to about 11.9 microns, or from about 0.0003 microns to about 11.8 microns, or from about 0.0004 microns to about 11.7 microns, or from about 0.0005 microns to about 11.6 microns, or from about 0.0006 microns to about 11.5 microns, or from about 0.0007 microns to about 11.4 microns, or from about 0.0008 microns to about 11.3 microns, or from about 0.0009 microns to about 11.2 microns, or from about 0.001 microns to about 11.1 microns, or from about 0.0011 microns to about 11 microns, or from about 0.0012 microns to about 10.9 microns, or from about 0.0013 microns to about 10.8 microns, or from about 0.0014 microns to about 10.7 microns, or from about 0.0015 microns to about 10.6 microns, or from about 0.0016 microns to about 10.5 microns, or from about 0.0017 microns to about 10.4 microns, or from about 0.0018 microns to about 10.3 microns, or from about 0.0019 microns to about 10.2 microns, or from about 0.002 microns to about 10.1 microns, or from about 0.0021 microns to about 10 microns, or from about 0.0022 microns to about 9.9 microns, or from about 0.0023 microns to about 9.8 microns, or from about 0.0024 microns to about 9.7 microns, or from about 0.0025 microns to about 9.6 microns, or from about 0.0026 microns to about 9.5 microns, or from about 0.0027 microns to about 9.4 microns, or from about 0.0028 microns to about 9.3 microns, or from about 0.0029 microns to about 9.2 microns, or from about 0.003 microns to about 9.1 microns, or from about 0.0031 microns to about 9 microns, or from about 0.0032 microns to about 8.9 microns, or from about 0.0033 microns to about 8.8 microns, or from about 0.0034 microns to about 8.7 microns, or from about 0.0035 microns to about 8.6 microns, or from about 0.0036 microns to about 8.5 microns, or from about 0.0037 microns to about 8.4 microns, or from about 0.0038 microns to about 8.3 microns, or from about 0.0039 microns to about 8.2 microns, or from about 0.004 microns to about 8.1 microns, or from about 0.0041 microns to about 8 microns, or from about 0.0042 microns to about 7.9 microns, or from about 0.0043 microns to about 7.8 microns, or from about 0.0044 microns to about 7.7 microns, or from about 0.0045 microns to about 7.6 microns, or from about 0.0046 microns to about 7.5 microns, or from about 0.0047 microns to about 7.4 microns, or from about 0.0048 microns to about 7.3 microns, or from about 0.0049 microns to about 7.2 microns, or from about 0.005 microns to about 7.1 microns, or from about 0.0051 microns to about 7 microns, or from about 0.0052 microns to about 6.9 microns, or from about 0.0053 microns to about 6.8 microns, or from about 0.0054 microns to about 6.7 microns, or from about 0.0055 microns to about 6.6 microns, or from about 0.0056 microns to about 6.5 microns, or from about 0.0057 microns to about 6.4 microns, or from about 0.0058 microns to about 6.3 microns, or from about 0.0059 microns to about 6.2 microns, or from about 0.006 microns to about 6.1 microns, or from about 0.0065 microns to about 6 microns, or from about 0.007 microns to about 5.9 microns, or from about 0.0075 microns to about 5.8 microns, or from about 0.008 microns to about 5.7 microns, or from about 0.0085 microns to about 5.6 microns, or from about 0.009 microns to about 5.5 microns, or from about 0.0095 microns to about 5.4 microns, or from about 0.01 microns to about 5.3 microns, or from about 0.015 microns to about 5.2 microns, or from about 0.02 microns to about 5.1 microns, or from about 0.025 microns to about 5 microns, or from about 0.03 microns to about 4.9 microns, or from about 0.035 microns to about 4.8 microns, or from about 0.04 microns to about 4.7 microns, or from about 0.045 microns to about 4.6 microns, or from about 0.05 microns to about 4.5 microns, or from about 0.055 microns to about 4.4 microns, or from about 0.06 microns to about 4.3 microns, or from about 0.065 microns to about 4.2 microns, or from about 0.07 microns to about 4.1 microns, or from about 0.075 microns to about 4 microns, or from about 0.08 microns to about 3.9 microns, or from about 0.085 microns to about 3.8 microns, or from about 0.09 microns to about 3.7 microns, or from about 0.095 microns to about 3.6 microns, or from about 0.1 microns to about 3.5 microns, or from about 0.15 microns to about 3.4 microns, or from about 0.2 microns to about 3.3 microns, or from about 0.25 microns to about 3.2 microns, or from about 0.3 microns to about 3.1 microns, or from about 0.35 microns to about 3 microns, or from about 0.4 microns to about 2.9 microns, or from about 0.45 microns to about 2.8 microns, or from about 0.5 microns to about 2.7 microns, or from about 0.55 microns to about 2.6 microns, or from about 0.6 microns to about 2.5 microns, or from about 0.65 microns to about 2.4 microns, or from about 0.7 microns to about 2.3 microns, or from about 0.75 microns to about 2.2 microns, or from about 0.8 microns to about 2.1 microns, or from about 0.85 microns to about 2 microns, or from about 0.9 microns to about 1.9 microns, or from about 0.95 microns to about 1.8 microns, or from about 1 micron to about 1.7 microns, or from about 1.1 microns to about 1.6 microns, or from about 1.2 microns to about 1.5 microns, or from about 1.3 microns to about 1.4 microns, or even about 1.35 microns as measured at 50X via optical interferometry. Here, as well as elsewhere in the specification and claims, individual numerical values can be combined to form additional, or even new/non-disclosed, numerical ranges.

In still another embodiment, if the stopper of the present invention is partially, or fully, laminated the surface roughness of the laminated film, or the top most laminated film in the case where two or more films are used to provide an overall laminated coating, is independently in a range of about 0.0001 microns to about 6 microns, or from about 0.0002 microns to about 5.9 microns, or from about 0.0003 microns to about 5.8 microns, or from about 0.0004 microns to about 5.7 microns, or from about 0.0005 microns to about 5.6 microns, or from about 0.0006 microns to about 5.5 microns, or from about 0.0007 microns to about 5.4 microns, or from about 0.0008 microns to about 5.3 microns, or from about 0.0009 microns to about 5.2 microns, or from about 0.001 microns to about 5.1 microns, or from about 0.0011 microns to about 5 microns, or from about 0.0012 microns to about 4.9 microns, or from about 0.0013 microns to about 4.8 microns, or from about 0.0014 microns to about 4.7 microns, or from about 0.0015 microns to about 4.6 microns, or from about 0.0016 microns to about 4.5 microns, or from about 0.0017 microns to about 4.4 microns, or from about 0.0018 microns to about 4.3 microns, or from about 0.0019 microns to about 4.2 microns, or from about 0.002 microns to about 4.1 microns, or from about 0.0021 microns to about 4 microns, or from about 0.0022 microns to about 3.9 microns, or from about 0.0023 microns to about 3.8 microns, or from about 0.0024 microns to about 3.7 microns, or from about 0.0025 microns to about 3.6 microns, or from about 0.0026 microns to about 3.5 microns, or from about 0.0027 microns to about 3.4 microns, or from about 0.0028 microns to about 3.3 microns, or from about 0.0029 microns to about 3.2 microns, or from about 0.003 microns to about 3.1 microns, or from about 0.0031 microns to about 3 microns, or from about 0.0032 microns to about 2.9 microns, or from about 0.0033 microns to about 2.8 microns, or from about 0.0034 microns to about 2.7 microns, or from about 0.0035 microns to about 2.6 microns, or from about 0.0036 microns to about 2.5 microns, or from about 0.0037 microns to about 2.4 microns, or from about 0.0038 microns to about 2.3 microns, or from about 0.0039 microns to about 2.2 microns, or from about 0.004 microns to about 2.1 microns, or from about 0.0041 microns to about 2 microns, or from about 0.0042 microns to about 1.9 microns, or from about 0.0043 microns to about 1.8 microns, or from about 0.0044 microns to about 1.7 microns, or from about 0.0045 microns to about 1.6 microns, or from about 0.0046 microns to about 1.5 microns, or from about 0.0047 microns to about 1.4 microns, or from about 0.0048 microns to about 1.3 microns, or from about 0.0049 microns to about 1.2 microns, or from about 0.005 microns to about 1.1 microns, or from about 0.0051 microns to about 1 micron, or from about 0.0052 microns to about 0.9 microns, or from about 0.0053 microns to about 0.8 microns, or from about 0.0054 microns to about 0.7 microns, or from about 0.0055 microns to about 0.6 microns, or from about 0.0056 microns to about 0.5 microns, or from about 0.0057 microns to about 0.4 microns, or from about 0.0058 microns to about 0.3 microns, or from about 0.0059 microns to about 0.2 microns, or from about 0.006 microns to about 0.1 microns, or from about 0.0065 microns to about 0.095 microns, or from about 0.007 microns to about 0.09 microns, or from about 0.0075 microns to about 0.085 microns, or from about 0.008 microns to about 0.08 microns, or from about 0.0085 microns to about 0.075 microns, or from about 0.009 microns to about 0.07 microns, or from about 0.0095 microns to about 0.065 microns, or from about 0.01 microns to about 0.06 microns, or from about 0.015 microns to about 0.055 microns, or from about 0.02 microns to about 0.05 microns, or from about 0.025 microns to about 0.045 microns, or from about 0.03 microns to about 0.04 microns, or even 0.035 microns as measured at 50X via optical interferometry. Here, as well as elsewhere in the specification and claims, individual numerical values can be combined to form additional, or even new/non-disclosed, numerical ranges.

In the embodiment where the syringe barrel is formed from glass, the compression ratio of the stopper versus the glass barrel inner diameter is from about 1 percent to about to about 10 percent, or from about 1.25 percent to about 9.75 percent, or from about 1.5 percent to about 9.5 percent, or from about 1.75 percent to about 9.25 percent, or from about 2 percent to about 9 percent, or from about 2.25 percent to about 8.75 percent, or from about 2.5 percent to about 8.5 percent, or from about 2.75 percent to about 8.25 percent, or from about 3 percent to about 8 percent, or from about 3.25 percent to about 7.75 percent, or from about 3.5 percent to about 7.5 percent, or from about 3.75 percent to about 7.25 percent, or from about 4 percent to about 7 percent, or from about 4.25 percent to about 6.75 percent, or from about 4.5 percent to about 6.5 percent, or from about 4.75 percent to about 6.25 percent, or from about 5 percent to about 6 percent, or from about 5.25 percent to about 5.75 percent, or even about 5.5 percent as determined from nominal dimensions meaning maximum outer diameter of the stopper in free state versus minimum sealing barrel inner diameter (consider in some instances that there may be a draft on the barrel inner diameter). Here, as well as elsewhere in the specification and claims, individual numerical values can be combined to form additional, or even new/non-disclosed, numerical ranges.

In one embodiment, the contact pressure generated at the sealing surface (be it glass, polymer, metal or another type of material that forms the surface to be sealed) by a stopper formed from a polymeric material in accordance with the present invention as measured within about a 0.01 mm width is more than about 90 percent of the indentation modulus, or yield strength, of a corresponding film of the polymeric material used to form such a stopper. As is known, the indentation modulus describes the elastic surface behavior of a material, in this case a polymeric material, during indentation. In another embodiment, the contact pressure generated at the sealing surface as measured within about a 0.01 mm width is more than about 91 percent, more than about 92 percent, more than about 93 percent, more than about 94 percent, more than about 95 percent, or even more than about 96 percent of the indentation modulus, or yield strength, of a corresponding film of the polymeric material used to form such a stopper. Here, as well as elsewhere in the specification and claims, individual numerical values can be combined to form additional, or even new/non-disclosed, numerical ranges. Alternatively, any of the contact pressure values noted above can be determined by comparing same to the contact pressure of a desired polymeric material at 50 percent elongation modulus.

In one embodiment, the polymeric material that is used to form a stopper of the present invention has a rubber substrate modulus in a range of about 4 MPa to about 8 MPa, or from about 4.25 MPa to about 7.75 MPa, or from about 4.5 MPa to about 7.5 MPa, or from about 4.75 MPa to about 7.25 MPa, or from about 5 MPa to about 7 MPa, or from about 5.25 MPa to about 6.75 MPa, or from about 5.5 MPa to about 6.5 MPa, or from about 5.75 MPa to about 6.25 MPa, or even about 6 MPa as measured at the limit of the stress versus strain of a molded part formed from a desired polymeric material without a laminated film thereon. Here, as well as elsewhere in the specification and claims, individual numerical values can be combined to form additional, or even new/non-disclosed, numerical ranges.

In one embodiment, it should be noted that due to the polymeric material used to form the stoppers of the present invention the sealing capabilities of such stoppers only changes in a linear relationship with contact width (Poiseuille equation). As such, the main aspect related to contact width is if in any continuous region along the seal percolation is reached. Accordingly, in one embodiment the ratio of the real contact area (Aᵣₑₐₗ) to the nominal contact area (Aₒ) - that is Aᵣₑₐₗ/Aₒ - is about 0.42 or more, or about 0.43 or more, or about 0.44 or more, or about 0.45 or more, or about 0.46 or more, or about 0.47 or more, or about 0.48 or more, or about 0.49 or more, or about 0.50 or more, or about 0.51 or more, or about 0.52 or more, or about 0.53 or more, or about 0.54 or more, or about 0.55 or more, or about 0.56 or more, or about 0.57 or more, or about 0.58 or more, or about 0.59 or more, or even about 0.60 or more. In another instance Aᵣₑₐₗ/Aₒ is about 0.61 or more, or about 0.62 or more, or about 0.63 or more, or about 0.64 or more, or about 0.65 or more, or about 0.66 or more, or about 0.67 or more, or about 0.68 or more, or about 0.69 or more, or even about 0.70 or more. Here, as well as elsewhere in the specification and claims, individual numerical values can be combined to form additional, or even new/non-disclosed, numerical ranges.

In one embodiment, the present invention uses various correlations between stopper geometry, roughness, indentation modulus of the surfaces, and/or modulus of substrate materials to achieve sealing (percolation threshold) that is independent of the surfaces energies of the sealing surfaces. As such, in one embodiment, the stoppers of the present invention are partially laminated and therefore each stopper has an elastomeric body, one or more fluoropolymer layers, and one or more (or even two or more ribs) laminated with the one or more fluoropolymer layers. In another embodiment, the stoppers of the present invention are fully laminated with all of the stopper being laminated with one or more fluoropolymer layers. In one embodiment, the one or more fluoropolymer layers described herein may include a single layer of densified expanded polytetrafluoroethylene (ePTFE), or the one or more fluoropolymer layers may include a composite fluoropolymer film having a barrier layer and a porous layer, the barrier layer including densified ePTFE, polytetrafluoroethylene (PTFE), fluorinated ethylene propylene (FEP), polyethylene (e.g., UHMWPE), polypropylene (e.g., syndiotactic-PP), polyvinylidene fluoride, polyvinylfluoride, perfluoropropylevinylether, a perfluoroalkoxy polymer, polysulfone, and copolymers and combinations thereof. In another embodiment, any high temperature material, such as a polymeric material, can be used to form the one or more lamination layers. Suitable non-fluoropolymer materials for such lamination layers include, but are not limited to, UHMWPE, syndiotactic-polypropylene blends, and/or polysulfone.

The following is one non-limiting example of a material for the formation of stoppers in accordance with the present invention. It should be noted that the present invention is not limited solely to this one example but rather is to be broadly construed given the various parameters and properties set forth herein.

A stopper in accordance with the present invention is formed to have a stopper body with one rib, two ribs, or even three or more ribs thereon, where at least one such rib is a sealing rib. The shape of such stopper is not critical and can be round, square, a composite shape, etc. The shape of the stopper is this example is such that the combination of the contact geometry-width, rubber modulus and roughness achieve along a continuous path along the sealing surface of any sealing element 90 percent or more of the indentation modulus of a corresponding film of the polymeric material used to form such a stopper (as defined in more detail above). The material used to form such a stopper is any rubber material described herein which achieves a Young's modulus of about 5 MPa to about 10 MPa, or even about 6 MPa to about 9 MPa (as defined in more detail above). In one embodiment, the material used to form the stopper of the present invention contains one or more suitable fillers, and can optionally even be a cross-linked polymeric material. Suitable filler materials for the polymeric materials include, but are not limited to, various silica compounds including silica compounds treated with one or more coupling agents (e.g., a silane coupling agent), single or multiple walled carbon nanotubes (CNT). Additionally, other suitable filler materials for the polymeric materials disclosed herein are known in the art and as such are omitted herein for the sake of brevity.

In another embodiment, the polymeric material that can be used to form a stopper in accordance with the present invention has a stress relaxation of between about 0 percent and about 35 percent as determined from initial contact max pressure. In still another embodiment, the polymeric material that is used to form a stopper of the present invention has a stress relaxation of between about 0 percent and about 30 percent, or between about 2.5 percent and about 27.5 percent, or between about 5 percent and about 25 percent, or between about 7.5 percent and about 22.5 percent, or between about 10 percent and about 20 percent, or between about 12.5 percent and about 17.5 percent, or even about 15 percent as determined from initial contact max pressure. Here, as well as elsewhere in the specification and claims, individual numerical values can be combined to form additional, or even new/non-disclosed, numerical ranges.

In one embodiment, the polymeric material for use in forming the stopper of the present invention is any suitable butyl rubber material, or other polymer or rubber material, having a Shore A hardness in a range of about 50 to about 85, or from about 51 to about 84, or from about 52 to about 83, or from about 53 to about 82, or from about 54 to about 81, or from about 55 to about 80, or from about 56 to about 79, or from about 57 to about 78, or from about 58 to about 77, or from about 59 to about 76, or from about 60 to about 75, or from about 61 to about 74, or from about 62 to about 73, or from about 63 to about 72, or from about 64 to about 71, or from about 65 to about 70, or from about 66 to about 69, or from about 67 to about 68, or even about 67.5. In another embodiment, the polymeric material for use in forming the stopper of the present invention is any suitable butyl rubber material having a Young's modulus in a range of about 5 MPa to about 10 MPa, or from about 5.1 MPa to about 9.9 MPa, or from about 5.2 MPa to about 9.8 MPa, or from about 5.3 MPa to about 9.7 MPa, or from about 5.4 MPa to about 9.6 MPa, or from about 5.5 MPa to about 9.5 MPa, or from about 5.6 MPa to about 9.4 MPa, or from about 5.7 MPa to about 9.3 MPa, or from about 5.8 MPa to about 9.2 MPa, or from about 5.9 MPa to about 9.1 MPa, or from about 6 MPa to about 9 MPa, or from about 6.1 MPa to about 8.9 MPa, or from about 6.2 MPa to about 8.8 MPa, or from about 6.3 MPa to about 8.7 MPa, or from about 6.4 MPa to about 8.6 MPa, or from about 6.5 MPa to about 8.5 MPa, or from about 6.6 MPa to about 8.4 MPa, or from about 6.7 MPa to about 8.3 MPa, or from about 6.8 MPa to about 8.2 MPa, or from about 6.9 MPa to about 8.1 MPa, or from about 7 MPa to about 8 MPa, or from about 7.1 MPa to about 7.9 MPa, or from about 7.2 MPa to about 7.8 MPa, or from about 7.3 MPa to about 7.7 MPa, or from about 7.4 MPa to about 7.6 MPa, or even about 7.5 MPa. In still another embodiment, the polymeric material for use in forming the stopper of the present invention is any suitable butyl rubber material having a Shore A hardness within one of the above ranges in conjunction with a Young's modulus within one of the above ranges. Here, as well as elsewhere in the specification and claims, individual numerical values can be combined to form additional, or even new/non-disclosed, numerical ranges.

In another embodiment, the polymeric material for use in forming the stopper of the present invention is any suitable butyl rubber material having a stress relaxation over a period of 24 hours better than about 20 percent at room temperature (RT), better than about 25 percent at RT, or even better than about 30 percent at RT. In still another embodiment, the polymeric material for use in forming the stopper of the present invention is any of the polymeric materials disclosed above that have a stress relaxation better than about 21 percent at room temperature (RT), better than about 22 percent at RT, better than about 23 percent at RT, better than about 24 percent at RT, better than about 25 percent at RT, better than about 26 percent at RT, better than about 27 percent at RT, better than about 28 percent at RT, better than about 29 percent at RT, or even better than about 30 percent at RT. Here, as well as elsewhere in the specification and claims, individual numerical values can be combined to form additional, or even new/non-disclosed, numerical ranges.

In still another embodiment, any of the polymeric materials disclosed for use in conjunction with the stoppers of the present invention can have any combination of two, three or more, or even four or more, of any of the various chemical and/or physical properties disclosed herein.

In one embodiment, the polymeric material for use in conjunction with the stoppers of the present invention are suitable butyl rubbers having a high Mooney viscosity, as defined above, that can be partially, or fully, laminated with one or more layers of PTFE, UHMWPE, or some other lamination material as detailed above which has a melting temperature that is within the rubber molding temperature parameters.

In one instance, a high Mooney viscosity bromo-butyl rubber composition is loaded with about 40 phr to about 90 phr reinforcing silica filler with a sulfur donor fast-accelerator type of curing package activated containing a metal oxide and stearic acid. In another instance, the loading range of this bromo-butyl rubber composition with the afore-mentioned silica filler is in a range of about 42 phr to about 88 phr, or from about 44 phr to about 86 phr, or from about 46 phr to about 84 phr, or from about 48 phr to about 82 phr, or from about 50 phr to about 80 phr, or from about 52 phr to about 78 phr, or from about 54 phr to about 76 phr, or from about 56 phr to about 74 phr, or from about 58 phr to about 72 phr, or from about 60 phr to about 70 phr, or from about 62 phr to about 68 phr, or from about 64 phr to about 66 phr, or even about 65 phr silica filler. As used herein, the term per hundred resin (phr) means the amount of an additive, resin, or some other component, by weight as calculated based on 100 parts by weight of base polymer, rubber, and/or resin. Here, as well as elsewhere in the specification and claims, individual numerical values can be combined to form additional, or even new/non-disclosed, numerical ranges.

Some alternative fillers are high reinforcement silica treated with a coupling agent such as a silane coupling agent, high surface area carbon based fillers such as furnace carbon blacks that comply with ASTM N110, N220, N330, N550, N660 standards, single wall and multi-wall carbon nano-tubes, graphene oxide nano-particles, phenolic and resol resin networks, and thermoplastics such as polypropylene. Additional rubber formulations for possible lamination with low melting temperature film (e.g., a suitable ultrahigh molecular weight polyethylene) are high Mooney viscosity bromo butyl rubber formulations having therein a high load (e.g., from about 60 phr to about 70 phr) reinforcing silica filler with a sulfur donor ultra-accelerator type of curing package (metal-organic complex) that has been activated with a metal oxide and a suitable carbamate-type secondary accelerator. In one embodiment the reinforcing silica filler is chosen from silica, sodium silicate, potassium silicate, calcium silicate, magnesium silicate, aluminum silicate. Additional suitable variants of this type of rubber formulation are those that utilize either a high reinforcement level (e.g., from about 60 phr to about 70 phr) of silica treated with a suitable coupling agent (e.g., a silane coupling agent), a dispersion polymer system, and/or a liquid rubber polymer additive (e.g., a suitable butadiene additive). Another variant of these type of rubber formulations utilize a high reinforcement level (e.g., from about 60 phr to 70 phr) of pre-treated silicates. The silica could be pretreated with a silane coupling agent, alkylammonium salts such as octadecylamine and octadecyltrimethylamine, and quaternary amines.

In one embodiment, a suitable filler according to the present invention is any of the filler materials disclosed herein that have a surface area greater than about 100 m²/g, or greater than about 110 m²/g, or greater than about 120 m²/g, or greater than about 130 m²/g, or greater than about 140 m²/g, or greater than about 150 m²/g, or greater than about 160 m²/g, or greater than about 170 m²/g, or greater than about 180 m²/g, or greater than about 190 m²/g, or greater than about 200 m²/g, or greater than about 210 m²/g, or greater than about 220 m²/g, or greater than about 230 m²/g, or greater than about 240 m²/g, or greater than about 250 m²/g, or greater than about 260 m²/g, or greater than about 270 m²/g, or greater than about 280 m²/g, or greater than about 290 m²/g, or even greater than about 300 m²/g. In another embodiment, a suitable filler according to the present invention is any of the filler materials disclosed herein that have a surface area greater than about 325 m²/g, or greater than about 350 m²/g, or greater than about 375 m²/g, or greater than about 400 m²/g, or greater than about 425 m²/g, or greater than about 450 m²/g, or greater than about 475 m²/g, or greater than about 500 m²/g, or greater than about 525 m²/g, or greater than about 550 m²/g, or greater than about 575 m²/g, or even greater than about 600 m²/g. Here, as well as elsewhere in the specification and claims, individual numerical values can be combined to form additional, or even new/non-disclosed, numerical ranges.

In an alternate embodiment the base polymeric material used in forming the stopper of the present invention is any suitable styrene-butadiene, high-cis butadiene, ethylene propylene diene terpolymer, brominated isobutylene paramethyl-styrene terpolymers, chloro or bromo isobutylene-isoprene co-polymer, or any blend thereof having a Shore A hardness in a range of about 60 to about 70, or from about 61 to about 69, or from about 62 to about 68, or from about 63 to about 67, or from about 64 to about 66, or even about 65. In an alternate embodiment one or more of these base polymers are blended with or without reinforcing fillers in combination with a curative package to obtain a Young's modulus in a range of about 5 MPa to about 8 MPa, or from about 5.1 MPa to about 7.9 MPa, or from about 5.2 MPa to about 7.8 MPa, or from about 5.3 MPa to about 7.7 MPa, or from about 5.4 MPa to about 7.6 MPa, or from about 5.5 MPa to about 7.5 MPa, or from about 5.6 MPa to about 7.4 MPa, or from about 5.7 MPa to about 7.3 MPa, or from about 5.8 MPa to about 7.2 MPa, or from about 5.9 MPa to about 7.1 MPa, or from about 6 MPa to about 7 MPa, or from about 6.1 MPa to about 6.9 MPa, or from about 6.2 MPa to about 6.8 MPa, or from about 6.3 MPa to about 6.7 MPa, or from about 6.4 MPa to about 6.6 MPa, or even about 6.5 MPa. In these embodiments acceptable cure packages could include sulfur donor fast-accelerator type of curing package activated containing a metal oxide and stearic acid, alternately, organic peroxides with or without type 1 and type 2 co-agents, and rubber maker sulfur in combination with thiazoles, sulfenamides, guanidines, and dithiocarbamates, individually or in unique combinations.

Given the above, in one embodiment the present invention is directed to a stopper comprising: a stopper body formed from a polymeric material; and at least one sealing rib formed on at least one portion of the stopper body, wherein the polymeric material is selected from one or more polyolefins, one or more polyamides, one or more polyesters, one or more polystyrenes, one or more polyurethanes, one or more polycarbonates, one or more acrylonitrile-butadiene-styrenes, one or more fluoropolymers, one or more polyacrylates, one or more elastomeric rubbers, one or more polyisoprenes, one or more polyisobutylene, one or more polybutadienes, one or more chloroprene rubbers, one or more butyl rubbers, one or more halogenated butyl rubbers, one or more styrene-butadiene rubbers, one or more nitrile rubbers, one or more hydrogenated nitrile rubbers, natural rubber, one or more ethylene propylene rubbers, one or more ethylene propylene diene rubbers, or blends of any two or more thereof that have a Young's modulus in a range of about 5 MPa to about 10 MPa and a Shore A hardness in a range of about 50 to 85.

In another embodiment, the present invention is directed to a stopper that is formed from a polymeric material that has a Young's modulus in a range of about 6 MPa to about 9 MPa and a Shore A hardness in a range of about 55 to 75. In still another embodiment, the present invention is directed to a stopper that is formed from a polymeric material that has a Mooney viscosity of greater than about 40. In still another embodiment, the present invention is directed to a stopper that is formed from a polymeric material that has a stress relaxation better than about 30 percent at room temperature (RT) in a period of 24 hours. In still another embodiment, the present invention is directed to a stopper that is formed from a polymeric material that further comprises at least one filler present at an amount in a range of about 40 phr to about 90 phr, or more preferably in a range of about 60 phr to about 70 phr. In still another embodiment, the present invention is directed to a stopper that is formed from a polymeric material that has a filler is selected from a silica filler. In still another embodiment, the present invention is directed to a stopper that is formed from a polymeric material that is selected from a bromo butyl rubber.

In still another embodiment, the present invention is directed to a stopper that has a stopper body that has an Aᵣₑₐₗ/Aₒ in a range of about 0.42 to about 0.70. In still another embodiment, the present invention is directed to a stopper where the stopper body further comprises at least two ribs formed thereon. In still another embodiment, the present invention is directed to a stopper where at least one of the ribs is laminated with at least one lamination layer. In still another embodiment, the present invention is directed to a stopper where all of the ribs are laminated with at least one lamination layer. In still another embodiment, the present invention is directed to a stopper where all of the ribs and the stopper body are laminated with at least one lamination layer. In still another embodiment, the present invention is directed to a stopper where any lamination layer is formed from at least one compound selected form densified expanded polytetrafluoroethylene (ePTFE), polytetrafluoroethylene (PTFE), fluorinated ethylene propylene (FEP), polyethylene, polypropylene, polyvinylidene fluoride, polyvinylfluoride, perfluoropropylevinylether, a perfluoroalkoxy polymer, polysulfone, and copolymers and combinations thereof. In still another embodiment, the present invention is directed to a stopper where any lamination layer is formed from at least one layer of polytetrafluoroethylene (PTFE) or UHMWPE. In still another embodiment, the present invention is directed to a stopper where the surface roughness of the polymeric material that is used to form the stopper of the present invention is in a range of about 0.001 microns to about 4 microns as measured at 50X via optical interferometry.

In still another embodiment, the present invention is directed to a sealing device comprising: a sealing body formed from a polymeric material; and at least one sealing structure and/or or sealing rib formed on at least one portion of the sealing body, wherein the polymeric material is selected from one or more polyolefins, one or more polyamides, one or more polyesters, one or more polystyrenes, one or more polyurethanes, one or more polycarbonates, one or more acrylonitrile-butadiene-styrenes, one or more fluoropolymers, one or more polyacrylates, one or more elastomeric rubbers, one or more polyisoprenes, one or more polyisobutylene, one or more polybutadienes, one or more chloroprene rubbers, one or more butyl rubbers, one or more halogenated butyl rubbers, one or more styrene-butadiene rubbers, one or more nitrile rubbers, one or more hydrogenated nitrile rubbers, natural rubber, one or more ethylene propylene rubbers, one or more ethylene propylene diene rubbers, or blends of any two or more thereof that have a Young's modulus in a range of about 5 MPa to about 10 MPa and a Shore A hardness in a range of about 50 to 85, that can be utilized to seal any type of medical device and/or other device needing an adequate sealing device.

Given the above, in another embodiment the present invention is directed to a syringe comprising: a syringe barrel; a plunger, wherein the plunger is designed to fit within the syringe barrel; and a stopper according to any of the embodiments disclosed herein, wherein the stopper is adapted for attachment to one end of the plunger so as act as a seal for the syringe barrel.

While this disclosure has been described as having exemplary designs, the present disclosure can be further modified within the spirit and scope of this disclosure. This application is therefore intended to cover any variations, uses, or adaptations of the disclosure using its general principles. Further, this application is intended to cover such departures from the present disclosure that are known or customarily practiced in the art to which this disclosure pertains and which fall within the limits of the appended claims.

## Claims

1. A stopper comprising:
a stopper body formed from a polymeric material; and
at least one sealing rib formed on at least one portion of the stopper body,
wherein the polymeric material is selected from one or more polyolefins, one or more polyamides, one or more polyesters, one or more polystyrenes, one or more polyurethanes, one or more polycarbonates, one or more acrylonitrile-butadiene-styrenes, one or more fluoropolymers, one or more polyacrylates, one or more elastomeric rubbers, one or more polyisoprenes, one or more polyisobutylene, one or more polybutadienes, one or more chloroprene rubbers, one or more butyl rubbers, one or more halogenated butyl rubbers, one or more styrene-butadiene rubbers, one or more nitrile rubbers, one or more hydrogenated nitrile rubbers, natural rubber, one or more ethylene propylene rubbers, one or more ethylene propylene diene rubbers, or blends of any two or more thereof where the polymeric material, or the blend of polymeric materials, has a Young's modulus in a range of about 5 MPa to about 10 MPa and a Shore A hardness in a range of about 50 to about 85.

2. The stopper of claim 1, wherein the polymeric material has a Young's modulus in a range of about 5 MPa to about 8 MPa and a Shore A hardness in a range of about 60 to about 70.

3. The stopper of any of claims 1 or 2, wherein the polymeric material has a Mooney viscosity of greater than about 40, before curing.

4. The stopper of any of claims 1 to 3, wherein the polymeric material has a stress relaxation better than about 30 percent at room temperature (RT) in a period of 24 hours and a compression ratio (CR) under 25 percent (as determined by ASTM D4065).

5. The stopper of any of claims 1 to 4, wherein the polymeric material further comprises at least one filler present at an amount in a range of about 50 phr to about 90 phr, or more preferably in a range of about 60 phr to about 70 phr.

6. The stopper of claim 5, wherein the filler is selected from any one or more suitable reinforcement fillers with a particle having a surface area greater than about 100 m²/g, or preferably a silica filler having a surface area greater than 100 m²/g.

7. The stopper of any of claims 1 to 6, wherein the polymeric material is selected from a halogenated isobutylene-isoprene copolymer rubber, and optionally, the polymeric material is selected from a styrene-butadiene, a high-cis butadiene, an ethylene propylene diene terpolymer, or a brominated isobutylene paramethyl-styrene terpolymer.

8. The stopper of any of claims 1 to 7, wherein the stopper body has an Aᵣₑₐₗ/Aₒ in the range of about 0.42 to about 0.70 on at least one sealing rib.

9. The stopper of any of claims 1 to 8, wherein the stopper body further comprises at least two sealing ribs formed thereon, and optionally, at least one of the sealing ribs is laminated with at least one lamination layer.

10. The stopper of any of claims 1 to 9, wherein all of the sealing ribs are laminated with at least one lamination layer.

11. The stopper of any of claims 1 to 10, wherein all of the sealing ribs and the stopper body are laminated with at least one lamination layer.

12. The stopper of any of claims 10 to 11, wherein the at least one lamination layer is formed from at least one compound selected from densified expanded polytetrafluoroethylene (ePTFE), polytetrafluoroethylene (PTFE), fluorinated ethylene propylene (FEP), a polyethylene such as a UHMWPE, polypropylene, polyvinylidene fluoride, polyvinylfluoride, perfluoropropylevinylether, a perfluoroalkoxy polymer, and copolymers and combinations thereof.

13. The stopper of any of claims 1 to 12, wherein the surface roughness of the polymeric material that is used to form the stopper of the present invention is in a range of about 0.0002 microns to about 4 microns as measured at 50X via optical interferometry.

14. A syringe comprising:
a syringe barrel;
a plunger, wherein the plunger is designed to fit within the syringe barrel, wherein the syringe barrel has a compression ratio (CR) of about 1 percent to about 7.4 percent where CR is defined as the initial value before assembly such that CR = 100% x (R_{free stopper OD} - R_{barrel ID}) / R_{barrel ID}; and
a stopper according to any of claims 1 to 16, wherein the stopper is adapted for attachment to one end of the plunger so as act as a seal for the syringe barrel.

15. A sealing device comprising:
a sealing body formed from a polymeric material; and
at least one sealing structure and/or or sealing rib formed on at least one portion of the sealing body,
wherein the polymeric material is selected from one or more polyolefins, one or more polyamides, one or more polyesters, one or more polystyrenes, one or more polyurethanes, one or more polycarbonates, one or more acrylonitrile-butadiene-styrenes, one or more fluoropolymers, one or more polyacrylates, one or more elastomeric rubbers, one or more polyisoprenes, one or more polyisobutylene, one or more polybutadienes, one or more chloroprene rubbers, one or more butyl rubbers, one or more halogenated butyl rubbers, one or more styrene-butadiene rubbers, one or more nitrile rubbers, one or more hydrogenated nitrile rubbers, natural rubber, one or more ethylene propylene rubbers, one or more ethylene propylene diene rubbers, or blends of any two or more thereof where the polymeric material, or the blend of polymeric materials, has a Young's modulus in a range of about 5 MPa to about 10 MPa and a Shore A hardness in a range of about 50 to about 85.
